# EUROPEAN PATENT APPLICATION

(11) **EP 4 700 111 A1**
(43) Date of publication of application: **25.02.2026**
(21) Application number: 24306393.0
(22) Date of filing: 23.08.2024
(51) Int. Cl.: C12M 3/00, C12M 1/00, C12M 1/26

(54) **BIOREACTOR SYSTEM**

(71) Applicant: Ispiron, 10190 Bucey-en-Othe (FR)
(72) Inventor: SAID, Ahmed, 91000 Évry-Courcouronnes (FR)
(74) Representative: Icosa

(57) **Abstract**

The present disclosure relates to a bioreactor for cell culturing, the bioreactor comprising: a culturing tank, a sliding filter, an extraction device configured to extract medium out of the culturing tank, a filling device configured to inject fresh medium in the culturing tank, and an agitating device.

## Description

### FIELD OF INVENTION

The present invention relates to a bioreactor for cell culturing.

### BACKGROUND OF INVENTION

Bioreactors are now extensively used for culturing cells *in vitro.* After culturing, cells may be extracted from the bioreactor to be lysed.

However, the known bioreactors are not satisfactory.

Indeed, the known bioreactors are only used for cell culturing. Therefore, cells must be precultured in a specific system before being transferred in the bioreactor for culturing. During the development of cells, the cultured cells must be transferred in larger bioreactors to follow the increase of volume of the cultured cells. Then, cells are transferred in a centrifuge to be lysed. This leads to several manipulations therefore increasing the risk of contamination by breaking sterility. Moreover, a laboratory technician is needed all along the process.

Moreover, for bioproduction, there is a need for very large volume such as several hundreds to several thousands of liters. To contain these large volumes, the dimensions of the bioreactors for bioproduction are very large. Therefore, these bioreactors cannot be disposed on a lab bench. Moreover, because of the large volume of fluid, there is a need of large quantity of active ingredients to reach the needed concentration. Some of these ingredients are expensive leading to very high cost to be spent for the cell culturing.

There is thus a need for a bioreactor which may be used on a lab bench for bioproduction, preferably in an automatic manner or a bioreactor which may be used from the seeding to the lysis of the cultured cells.

### SUMMARY

This disclosure thus relates to a bioreactor for cell culturing, the bioreactor comprising:
- a culturing tank configured to receive at least one cell to be cultured in a medium, the culturing tank extending along a tank axis,
- a filter disposed in the culturing tank so that the filter separates the culturing tank into an upper compartment and a lower compartment, the filter being configured to confine the at least one cell in the lower compartment, the filter being configured to slide along the tank axis thereby modifying volumes of the upper compartment and the lower compartment,
- an extraction device in fluidic communication with the culturing tank, the extraction device being configured to extract the medium out of the upper compartment of the culturing tank,
- a filling device in fluidic communication with the culturing tank, the filling device being configured to inject fresh medium in the upper compartment of the culturing tank, and
- an agitating device disposed in the lower compartment and configured to agitate the medium, the agitating device being preferably configured to slide along the tank axis.

This bioreactor may be used for bioproduction of cells while the volume of the tank is small enough to be placed on a lab bench.

Indeed, the filter is configured to slide along the culturing tank to adapt the culture volume with the stage of cell culturing without need to transfer cells to larger volume reactors between the pre-culture, culture and scale-up stages. Moreover, the medium may be renewed or replaced thank to the extraction and filling devices. The renewing of the culture medium allows to reproduce the culturing conditions as it would be in a bioproduction system.

Moreover, the sliding of the filter allows to separate cells from the medium. This bioreactor thus offers a real industrial advantage, since it enables the separation of cultured cells from their medium, without volume limitation. Moreover, the culturing process may be automated thereby reducing human intervention or handling outside the bioreactor.

Moreover, the bioreactor reduces clogging and fouling thanks to cells' natural tendency to sediment in the lower compartment.

The bioreactor thus enables continuous production and harvesting of various biological compounds, such as recombinant proteins, metabolites, viruses and bacteriophages.

Reactor can also be used to increase cell biomass. This can be of high interest for Chimeric Antigenic Receptor - T (CAR-T) cells culture, especially in the context of (near) Point of Care production (Hospitals). Automated cell culture will increase safety while decreasing time from biopsy to injection.

According to an advantageous embodiment, the bioreactor further comprises an actuator configured to slide the filter along the tank axis.

According to an advantageous embodiment, the culturing tank has a capacity ranging from 50 milliliters to 30 liters, preferably ranging from 1 liter to 25 liters, more preferably ranging from 5 liters to 20 liters.

This allows to place the tank on a lab bench.

According to one advantageous embodiment, the extraction device is configured to extract the medium out of the upper compartment when the upper compartment has a volume of more than twice a volume of the lower compartment, preferably more than four times the volume of the lower compartment, even more preferably more than 9 times the volume of the lower compartment.

According to one advantageous embodiment, the filter is hydrophilic.

According to one advantageous embodiment, the filter comprises pores having a size ranging from 10 kDa to 300 µm.

This allows to culture cells in suspension such as Chinese hamster ovary (CHO) cells, CAR-T cells, adhering cells to microcarrier supports, yeasts or bacteria.

According to one advantageous embodiment, the filling device is configured to inject the fresh medium in the upper compartment when the upper compartment has a volume of more than twice a volume of the lower compartment, preferably more than four times the volume of the lower compartment, even more preferably more than 9 times the volume of the lower compartment.

According to one advantageous embodiment, the bioreactor further comprises an electroporation device in fluidic communication with the culturing tank.

This allows to replace the use of chemical reagents during cell transfection. Chemical transfection is less efficient and more toxic than electroporation. The addition of the electroporation device therefore allows to increase culture yields.

According to one advantageous embodiment, the bioreactor further comprises a density measurement device in optical contact with the lower compartment, the density measurement device being configured to measure the density of the cultured cells, preferably the density measurement device is an optical device having a working wavelength ranging from 400 nm to 800 nm.

The density measurement device enables continuous monitoring of cell numbers, so that the filter height can be adjusted and/or nutrient addition can be controlled. The density measurement device may cooperate with pH or temperature sensors to enable the computer system to automatically adjust culture parameters.

According to one advantageous embodiment, the bioreactor further comprises at least one sensor configured to measure at least one parameter of the medium, the measured parameter being preferably a temperature or a pH.

The sensors thus allow to measure values representative of the culture parameters that can be adjusted if necessary.

According to one advantageous embodiment, the bioreactor further comprises a controller configured to control at least one physical or chemical parameter according to the parameter measured by the at least one sensor.

According to one advantageous embodiment, the bioreactor further comprises a sampling device configured to collect samples in the culturing tank.

According to one advantageous embodiment, the bioreactor further comprises a lysing device configured to lyse the cultured cells.

According to one advantageous embodiment, the lysing device comprises an ultrasonic device.

The ultrasonic device may be used for cell lysis by sonication. Advantageously, the same ultrasonic device may also allow to prevent filter clogging during culture.

This disclosure also relates to a method for cell culturing the method comprising:
- providing a bioreactor for cell culturing as described above,
- disposing at least one cell to be cultured in the culturing tank comprising a medium,
- agitating the medium with the agitating device,
- after a predetermined culturing time, refreshing the medium by:
   ∘ sliding the filter from a position wherein the upper compartment has a volume of more than one tenth of a volume of the lower compartment to decrease the volume of the lower compartment until the volume of the lower compartment is lower than one fourth of the volume of the upper compartment,
   ∘ extracting, with the extraction device, the medium out of the upper compartment,
   ∘ injecting, with the filling device, a fresh medium in the upper compartment, and
   ∘ sliding the filter to decrease the volume of the upper compartment so that a density of the cultured cells in the lower compartment is lower than a predetermined target expansion density while maintaining the lower compartment filled.

According to one advantageous embodiment, the medium comprised in the culturing tank comprises at least one growing agent and the fresh medium comprises at least one growing agent.

According to one advantageous embodiment, the medium filling the lower compartment comprises at least one growing agent and the fresh medium comprises at least one transfection agent.

According to one advantageous embodiment, the method further comprises:
- transfecting at least part of the cultured cells immersed in a medium comprising at least one transfection agent, transfecting comprising:
   ∘ sliding the filter to decrease the volume of the lower compartment until the volume of the lower compartment is lower than one fourth of the volume of the upper compartment,
   ∘ extracting, with the extraction device, the medium out of the upper compartment,
   ∘ injecting, with the filling device, a conditioning buffer in the upper compartment,
   ∘ sliding the filter to decrease the volume of the upper compartment,
   ∘ sliding the filter to decrease the volume of the lower compartment until the volume of the lower compartment is lower than one fourth of the volume of the upper compartment,
   ∘ extracting, with the extraction device, the medium out of the upper compartment, and
   ∘ performing an electroporation by generating at least one electrical pulse on the at least part of the cultured cells.

According to one advantageous embodiment, the method further comprises, after disposing at least one cell and before refreshing the medium, sliding the filter to decrease the volume of the upper compartment so that a density of the cultured cells in the lower compartment is lower than a predetermined target expansion density while maintaining the lower compartment filled.

### BRIEF DESCRIPTION OF THE DRAWINGS

**Figure 1** is an exploded perspective view (left) and exploded side view (right) of the bioreactor according to one embodiment wherein the actuator is disposed inside the tank.
**Figure 2** is a perspective view (left) and side view (right) of the assembled bioreactor of figure 1.
**Figure 3** is a perspective view (left) and side view (right) of the bioreactor according to one embodiment wherein the actuator is disposed outside the tank.

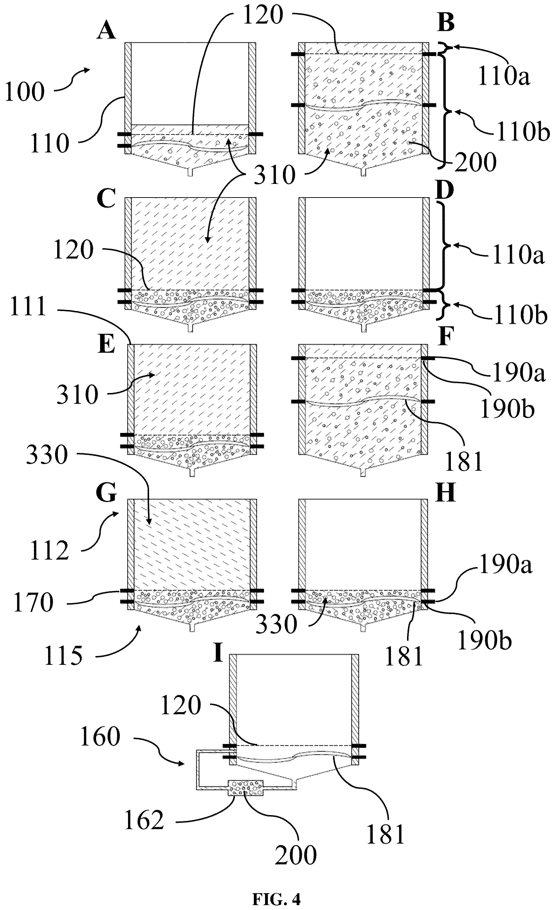

**Figure 4** comprises representations of the bioreactor according to one embodiment at different stages of cells culturing. **Figure 4A** is pre-culture stage. **Figure 4B** is culture stage. **Figure 4C** to **Figure 4E** are renewing of the culture medium by compressing cells in the lower compartment (Figure 4C), extracting the culture medium (Figure 4D) and filling the upper compartment with fresh culture medium (Figure 4E). **Figure 4F** is the continuation of cells culturing with the fresh culture medium. **Figure 4G** to **Figure 4I** are electroporation stage by compressing cells in the lower compartment and replacing the culture medium of the upper compartment by the conditioning buffer (Figure 4G), mixing cells in the conditioning buffer to wash the cells before extracting the mixed medium from the upper compartment (Figure 4H) and electroporate cells (Figure 4I).

### DETAILED DESCRIPTION

This disclosure relates to a bioreactor 100 for cell culturing.

The bioreactor 100 allows culturing all types of cells 200. For example, the bioreactor 100 allows culturing of mammalian cells, yeast or bacteria. Mammalian cells can be non-adherent, such as CHO cells, CAR-T cells or adherent cells on microcarrier supports, such as HEK293 or Hela cells.

Cells 200 are cultured in a medium, also named culture medium. The medium may be a liquid. The medium may comprise at least one nutrient (growing agent) such as amino acid, carbohydrate, vitamin, mineral salt, lipid, dissolved gas such as oxygen or carbon dioxide, growth factors, hormones, buffer, serum, water or the combination thereof. The medium may comprise at least one transfection agent, *i.e.,* exogeneous genetic material such as nucleic acids to be introduced into cells 200 during a transfection or a transduction process. The nucleic acids to be introduced can be plasmid DNA, siRNA or mRNA. The medium may comprise at least one chemical agent to perform chemical transfection. The chemical agent is configured to facilitate the uptake of the exogeneous genetic material by cells 200. The chemical agent is preferably calcium phosphate, lipofection reagents, or polyethylenimine. The medium may comprise viruses to perform viral transduction. The medium may comprise a conditioning buffer. Preferably, the conditioning buffer comprises no salts nor DNA.

The medium may comprise any combination of at least two of the culturing agent, the transfection agent, the chemical agent and the conditioning buffer.

The medium is intended to be refreshed at least once during cell culturing. The medium is refreshed by replacement of at least part of medium into which the cells are deposited by fresh medium. The fresh medium may be of the same type of the replaced medium (*i.e.,* comprising the same culturing agent, transfection agent, chemical agent or conditioning buffer) or may comprise different agent(s). When dealing with dissolved gas, the medium may be refreshed by a liquid medium comprising said dissolved gases or by contact with gas.

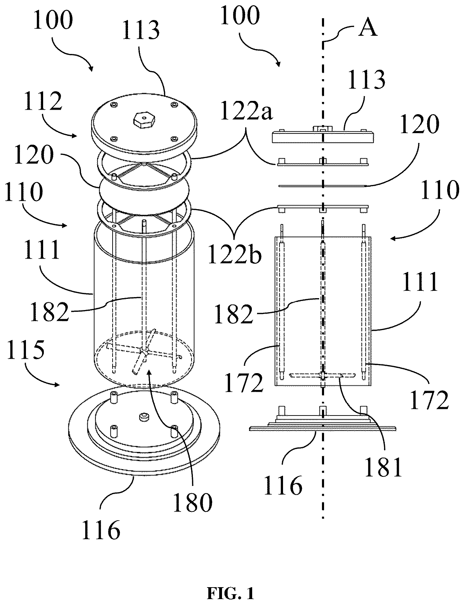

An example of bioreactor 100 is represented in figure 1, figure 2 and figure 3. The bioreactor 100 comprises:
- a culturing tank 110,
- a filter 120 disposed in the culturing tank 110, and
- a medium managing device.

The culturing tank 110 is configured to receive the medium and at least one cell 200 to be cultured in the medium. The culturing tank 110 extends along a tank axis A.

The culturing tank 110 has a general shape of a cylinder with preferably a circular section. The culturing tank 110 thus has, by definition, two extremities: a top 112 and a bottom 115. It will be appreciated that the terms "top"/"above"/"upper" and "bottom"/"below"/"lower" are utilized herein with reference to the orientation of the bioreactor in use along the tank axis A.

The dimensions of the section of the culturing tank 110 are measured perpendicularly to the tank axis A. Preferably, the dimensions of the section of the culturing tank 110 are constant along at least part the tank axis A so that the filter 120 may slide along said part of the tank axis A. A length of the culturing tank 110 is measured along the tank axis A between the top 112 and the bottom 115. The culturing tank may have a capacity lower than 30 liters, preferably lower than 25 liters, more preferably lower than 20 liters. The culturing tank may have a capacity larger than 50 milliliters, preferably larger than 0.5 liter, preferably larger than 1 liter, more preferably larger than 5 liters. This advantageously allows to place the culturing tank 110 on a lab bench. Despite this low capacity, the bioreactor 100 allows the bioproduction of cells thanks to the features described hereafter. Culturing tanks of small capacity (several tens of milliliters) allow to parallelize the cell culture in several culturing tanks connected in series or in parallel to the medium managing device.

The culturing tank 110 has an outer wall 111 separating an interior of the culturing tank 110 from an exterior of the culturing tank 110. The outer wall 111 thus has an outer surface and an inner surface. The culturing tank 110 is preferably hermetically sealed. The outer wall 111 may be transparent allowing, for example, visual inspection of the cell culture. The outer wall 111 may be opaque to preserve cells 200 and the medium from external light.

The top 112 of the culturing tank 110 may be closed, preferably hermetically closed, by a top cover 113. The bottom 115 of the culturing tank 110 may be closed, preferably hermetically closed, by a bottom cover 116. The bottom 115 may be planar as in figures 1-3 or may have a conical shape as in figure 4.

The filter 120 allows to separate the culturing tank 110 into an upper compartment 110a and a lower compartment 110b. To perform the separation of the compartments, the filter 120 preferably has dimensions corresponding to the dimensions of the section of the culturing tank 110. The separation is not totally hermetic. Indeed, the medium may be moved from one compartment to the other but cells 200 are confined in the lower compartment 110b. To do so, the filter 120 may be porous, *i.e.,* comprising at least one pore. Several pore sizes are possible. For example, the pore size is ranging from 10 kDa (corresponding to an ultra-filtration) to 300 µm. Preferably, for culturing CHO and yeast cells, the pore size is ranging from 0.45 µm to 10 µm. This size of pores allows the viruses injected into the upper compartment 110a for transduction to pass through the filter 120 to reach the lower compartment 110b. Preferably, for culturing cells adhering to microcarrier supports, the pore size is ranging from 10 µm to 300 µm. This size of pores also allows the viruses to pass through the filter 120. Preferably, for culturing bacteria, the pore size is ranging from 0.1 µm to 1 µm.

The filter 120 may be hydrophilic. For example, the filter 120 is made of cellulose, cellulose derivatives, ceramic such as zinc-titanium alloy, or any other biologically and mechanically compatible material.

The filter may be replaceable. For example, the filter 120 is disposed on a filter holder. More specifically, the filter 120 may be maintained between an upper part 122a of the filter holder and a lower part 122b of the filter holder.

The filter 120 is slidingly disposed in the culturing tank 100. In other words, the filter 120 is configured to slide along the tank axis A on at least part of the length of the culturing tank 110. The sliding of the filter 120 leads to a modification of volumes of the upper compartment 110a and the lower compartment 110b. Indeed, when the filter 120 is sliding towards the top 112 of the culturing tank 110, the volume of the upper compartment 110a is decreased and the volume of the lower compartment 110b is increased. Inversely, when the filter 120 is sliding towards the bottom 115 of the culturing tank 110, the volume of the upper compartment 110a is increased and the volume of the lower compartment 110b is proportionally decreased.

Advantageously, reducing the volume of the lower compartment 110b allows to increase the density of cells 200. This leads to a cells 200 separation without need of a centrifugation step.

To actuate the sliding of the filter 120, the bioreactor 100 may comprise an actuator 170 configured to slide the filter 120 along the tank axis A. The actuator 170 may be actuated manually or electronically. When the actuator 170 is actuated electronically, the actuator 170 is preferably actuated automatically.

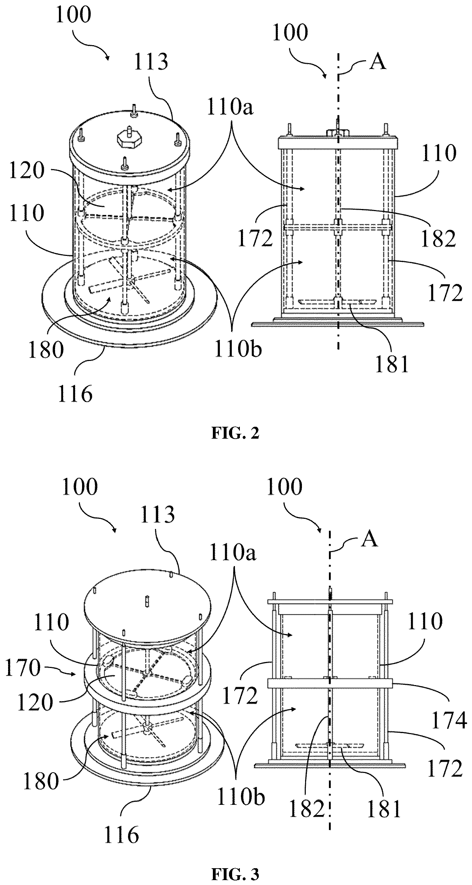

In an embodiment, the actuator 170 comprises at least one helical thread 172 such as a worm gear (or Archimedean screw). The helical thread 172 may be disposed inside the culturing tank 110 parallelly to the tank axis A as represented in figure 1 and figure 2. The filter 120 or the filter holder may comprise a nut corresponding to the helical thread 172. By rotating the helical thread 172, the filter 120 slides along the tank axis A. Alternatively, the helical thread 172 may be disposed outside the culturing tank 110 parallelly to the tank axis A. Each helical thread 172 may comprise a nut comprising a magnet while the filter 120 or the filter holder comprises the corresponding magnetic elements. Therefore, by rotating the helical threads 172, the nuts slide along the tank axis A. Thanks to the magnetic attraction acting through the outer wall 110 of the culturing tank 110, the filter 120 follows the sliding of the nuts comprising the magnets. Alternatively, an external ring 174 disposed outside the culturing tank 110 may cooperate with the helical threads 172 as represented in figure 3. The filter 120 or the filter holder comprises magnetic elements 190b corresponding to magnets 190a disposed on the external ring 174 as represented in figure 4. Therefore, by rotating the helical threads 172, the external ring 174 slides along the tank axis A. Thanks to the magnetic attraction acting through the outer wall 111 of the culturing tank 110, the filter 120 follows the sliding of the external ring 174 comprising the magnets 190a.

In another embodiment, the actuator 170 comprises a pinion (or gearwheel) meshes with a rack (or toothed bar) attached to the filter 120 or the filter holder, enabling to slide the filter along the culturing tank 110.

In another embodiment, the actuator 170 comprises a cable or belt wound around a pulley. The filter 120 or the filter holder is attached to the cable or the belt. Therefore, rotating the pulley leads to a sliding of the filter 120.

To avoid clogging of the filter 120, the bioreactor 100 may comprise an ultrasonic device which, when activated, is configured to generate ultrasounds allowing to unclog the filter 120 by vibration. The ultrasonic device may be piezoelectric, preferably made in ceramic. The ultrasonic device may be located above, preferably close to, the filter 120 in the upper compartment 110a. As the filter 120 descends towards the bottom of the culturing tank 110, pulses produced by the ultrasonic device clean the filter 120. The ultrasonic device is preferably in contact with the medium.

To refresh the medium, the medium managing device is configured to extract the medium from the culturing tank 110 and to inject fresh medium in the culturing tank 110. More specifically, the medium managing device is configured to extract the medium out of the upper compartment 110a of the culturing tank 110 and to inject fresh medium in the upper compartment 110a of culturing tank 110.

The medium managing device may comprise an extraction device configured to extract the medium out of the upper compartment of the culturing tank and a filling device configured to inject fresh medium in the upper compartment of the culturing tank 110.

For example, the extraction device comprises a pump fluidically connected to the culturing tank 110, preferably to the upper compartment 110a of the culturing tank 110. The pump may be actuated to suck and extract the medium from the culturing tank 110. For example, the pump may be connected to the culturing tank 110 via an extraction channel wherein a first extremity is connected to the pump and the other extremity is disposed in the upper compartment 110a. For example, the extraction channel is partially disposed in the upper compartment 110a via the top 112 of the culturing tank 110. When comprising a top cover 113, the extraction channel may be inserted through the top cover 113, preferably while maintaining the hermetic sealing. Alternatively, several outlets are disposed though the outer wall 111 along the length of the culturing tank 110. Each outlet is connected to the pump via the extraction channel and a valve. According to the height of the filter 120, at least one of the valves comprised in the upper compartment 110a is opened to allow the medium of the upper compartment 110a to be extracted by the pump.

The extraction device may be configured to extract the medium out of the upper compartment 110a when the upper compartment 110a has a volume of more than twice a volume of the lower compartment 110b so that the volume of the extracted medium is about 66% of the total capacity of the culturing tank 110. The extraction device may be configured to extract the medium out of the upper compartment 110a when the upper compartment 110a has a volume of more than four times the volume of the lower compartment 110b so that the volume of the extracted medium is about 80% of the total capacity of the culturing tank 110. Preferably, the extraction device may be configured to extract the medium out of the upper compartment 110a when the upper compartment 110a has a volume of more than nine times the volume of the lower compartment 110b so that the volume of the extracted medium is about 90% of the total capacity of the culturing tank 110.

For example, the filling device comprises at least one medium reservoir fluidically connected to the culturing tank 110, preferably to the upper compartment 110a of the culturing tank 110. The medium reservoir comprises the fresh medium. The filling device may be actuated to suck the fresh medium from at least one of the medium reservoirs and discharge the sucked medium in the culturing tank 110. The filling device may be configured to fill the culturing tank 110 up to its maximum capacity. Alternatively, the filling device may be configured to inject a predetermined volume of fresh medium in the culturing tank 100 as long as the maximum capacity is not reached. For example, each medium reservoir may be connected to the culturing tank 110 via a filling channel wherein a first extremity is disposed in the medium reservoir and the other extremity is disposed in the upper compartment 110a. For example, the filling channel is partially disposed in the upper compartment 110a via the top 112 of the culturing tank 110. When comprising a top cover 113, the filling channel may be inserted through the top cover 113, preferably while maintaining the hermetic sealing. Alternatively, several inlets are disposed though the outer wall 111 along the length of the culturing tank 110. Each inlet is connected to the medium reservoir via the filling channel and a valve. According to the height of the filter 120, at least one of the valves comprised in the upper compartment 110a is opened to allow fresh medium to be injected from the medium reservoir to the upper compartment 110a. When a plurality of medium reservoirs is used, the filling channel may comprise a general channel disposed in the culturing tank 100 and dividing into sub-channels disposed in each medium reservoir. Advantageously, a filter may be disposed at the extremity of the filling channel connected to the culturing tank 110. The diameter of the filling channel may be defined to provide a laminar flow of fresh medium.

In one embodiment, the inlets and the outlets are the same elements so that the medium is extracted and the fresh medium is injected via the same hole through the outer wall 111.

The filling device may be configured to inject fresh medium in the upper compartment 100a when the upper compartment 110a has a volume of more than twice the volume of the lower compartment 110b so that the volume of the injected medium is about 66% of the total capacity of the culturing tank 110. The filling device may be configured to inject fresh medium in the upper compartment 100a when the upper compartment 110a has a volume of more than four times the volume of the lower compartment 110b so that the volume of the injected medium is about 80% of the total capacity of the culturing tank 110. Preferably, the filling device may be configured to inject fresh medium in the upper compartment 100a when the upper compartment 110a has a volume of more than nine times the volume of the lower compartment 110b so that the volume of the injected medium is about 90% of the total capacity of the culturing tank 110.

The fresh medium, may comprise growing agent(s), transfection agent(s), chemical agent(s) or conditioning buffer or the combination thereof. The medium may be refreshed by contact with a gas.

Injecting fresh medium comprising growing agent(s) is advantageous. Indeed, during the cell growth, nutrients from the medium present in the culturing tank 110 are absorbed by the growing cells 200. Therefore, nutrients need to be injected to continue the cell growth.

For the transfection process, the bioreactor 100 may further comprises an electroporation device 160 allowing to introduce transfection agent(s) into cells 200. Electroporation is a technique which uses an electrical field applied on cells 200. The electrical pulses create temporary pores in the cell membrane, allowing transfection agent(s) to enter the cell. This method is highly efficient and can be used for a wide range of cell types, including bacterial, yeast, plant, and mammalian cells. The electroporation device allows to increase the cell culture efficiency because of the absence of chemical agent during the transfection process. However, the electroporation device may be used in combination with chemical agent(s). These chemical agent(s) advantageously allow to increase transfection yields by electroporation and/or increase cells survival.

The electroporation device 160 may be disposed inside the culturing tank 110, preferably inside the lower compartment 110b. The electroporation device 160 may be disposed outside the culturing tank 110. For example, the electroporation device 160 comprises an electroporation chamber 162 fluidically connected to the lower compartment 110b as represented in figure 4I. At the electroporation stage, cells 200 are moved in the electroporation chamber 162 and are injected back in the lower compartment 110b after the electroporation. This advantageously allows to electroporate cells 200 without extracting them from the culture medium and without contamination.

The bioreactor 100 may further comprise an agitating device 180 disposed in the lower compartment 110b. The agitating device 180 is configured to agitate the medium.

For example, the agitating device 180 may be an element disposed in the lower compartment 110b. For example, the agitating device 180 comprises beads, preferably magnetic beads moved in the lower compartment 110b by varying the magnetic field generated towards the magnetic beads. The beads may also be moved by a general movement of the culturing tank 110 such as an orbital, a linear or a circular movement generated by an agitating platform onto which the culturing tank 110 is disposed. In another example, the agitating device 180 is an impeller 181 comprising at least one blade, preferably four blades. The impeller 181 may be mounted on a shaft 182 configured to rotate the at least one blade as in figures 1-3. The rotation of the shaft 182 may be generated manually or electrically. When the rotation of the shaft 182 is generated electronically, the rotation of the shaft 182 is preferably generated automatically. Alternatively, bioreactor 100 further comprises at least one magnet 190a disposed outside the culturing tank 110 while the impeller 181 comprises the corresponding magnetic elements 190b as represented in figure 4. By rotating the at least one magnet 190a around the culturing tank 110, the magnetic attraction acting through the outer wall 111 implies a rotation of the blades.

The agitating device 180 mays also be configured to slide along the tank axis A on at least part of the height of lower compartment 110b.

To perform the sliding of the agitating device 180, the bioreactor 100 may comprise a second actuator configured to slide the agitating device 180 along the tank axis A. The second actuator may be actuated manually or electronically. When the second actuator is actuated electronically, the second actuator is preferably actuated automatically. The second actuator may be the actuator 170 configured to slide the filter 120. The agitating device 180 and the filter 120 may thus slide simultaneously and for example, in the same way. This advantageously reduces the space required compared to the use of two actuators.

In an embodiment, the second actuator comprises at least one helical thread such as a worm gear (or Archimedean screw). The helical thread may be disposed inside the culturing tank 110 parallelly to the tank axis A. The agitating device 180 may comprise a nut corresponding to the helical thread. By rotating the helical thread, the agitating device 180 slides along the tank axis A. The helical thread may be disposed outside the culturing tank 110 parallelly to the tank axis A. Each helical thread may comprise a nut comprising a magnet while the agitating device 180 comprises the corresponding magnetic elements. Therefore, by rotating the helical threads, the nuts slide along the tank axis A. Thanks to the magnetic attraction acting through the outer wall of the culturing tank 110, the agitating device 180 follows the sliding of the nuts comprising the magnets. Alternatively, an external ring disposed outside the culturing tank 110 may cooperate with the helical threads. The agitating device 180 comprises magnetic elements 190b corresponding to magnets 190a disposed on the external ring. Therefore, by rotating the helical threads, the external ring slides along the tank axis A. Thanks to the magnetic attraction acting through the outer wall 111 of the culturing tank 110, the agitating device 180 follows the sliding of the external ring comprising the magnets.

In another embodiment, the second actuator comprises a pinion (or gearwheel) meshes with a rack (or toothed bar) attached to the agitating device 180 allowing to slide the agitating device 180 along the culturing tank 110.

In another embodiment, the second actuator comprises a cable or belt wound around a pulley. The agitating device 180 is attached to the cable or the belt. Therefore, rotating the pulley leads to a sliding of the agitating device 180.

The bioreactor 100 may further comprise a lysing device configured to lyse the cultured cells 200. The lysing device may be configured to perform mechanical disruption. For example, the lysing device may be a homogenizer configured to apply shear forces on cells 200. In another example, the lysing device may comprise beads used to grind cells 200. In a preferred embodiment, the lysing device may be an ultrasonic device configured to generate ultrasonic waves to disrupt cell membranes. The ultrasonic device may be piezoelectric, preferably made in ceramic. The ultrasonic device may be located above, preferably close to, the filter 120 in the upper compartment 110a. The ultrasonic device may be in contact with the medium. The ultrasonic device may be disposed outside the culturing tank 110. The ultrasonic device may be the same as used to unclog the filter 120. For example, the frequency ranges used to unclog the filter 120 and to lyse the cells are different. The frequency ranges used to unclog the filter 120 and to lyse the cells may overlap. This advantageously reduces the space required compared to the use of two ultrasonic devices.

The lysing device may be configured to perform chemical disruption. The lysing device may comprise a lysing reservoir fluidically connected to the culturing tank 110, preferably to the lower compartment 110b of the culturing tank 110. The lysing reservoir may comprise detergents like Triton X-100, SDS, or NP-40 that solubilize the cell membrane by disrupting lipid-lipid and lipid-protein interactions, leading to cell lysis. This method is gentle and preserves protein functionality, making it ideal for protein extraction. The lysing reservoir may comprise enzymes like lysozyme (for bacterial cells) or trypsin (for animal cells) configured to degrade the cell wall or membrane, resulting in cell lysis. This method is gentle and preserves cellular components but requires specific enzymes for different cell types.

The lysing device may comprise a cooling device configured to perform freeze-thaw cycles by repeatedly freezing and thawing cells. This causes ice crystals to form, which puncture the cell membrane, leading to cell lysis. This method is simple and effective for lysing bacterial and mammalian cells but may not be suitable for all cell types due to potential protein denaturation.

The bioreactor may further comprise a sampling device configured to collect samples in the culturing tank. The sampling device can take the form of a pipe which is preferentially reversibly disposed in the lower compartment 110b so that it can be inserted in the lower compartment 110b to sample cells and then removed from the lower compartment 110b to extract the sampled cells. The filter 120 may be configured to be crossed by the pipe, for example at a sample port which is closed by a valve in absence of the pipe.

The bioreactor may further comprise a density measurement device configured to measure the density of the cultured cells 200. Indeed, the sliding of the filter 120 and/or the refreshing of medium are preferably performed according to the density of cells 200 as described here below. The density measurement device may be an optical device such as a spectrophotometer. The optical density measurement device may have a working wavelength ranging from 400 nm to 800 nm. The density measurement device may be disposed outside the culturing tank 110 or in the lower compartment 110b, preferably close to the filter 120; *i.e.,* closer to the filter 120 than the bottom of the culturing tank 110. The density measurement device is preferably disposed along the tank axis A. The density measurement device may be configured to measure values representative of the density of cells in the lower compartment 110b. Preferably, the density measurement device is moveable along the tank axis A in order to, for example, follow the sliding of the filter 120.

The bioreactor may further comprise at least one sensor configured to measure at least one parameter of the medium. For example, the sensor is configured to measure a value representative of the temperature, the pH or the ionic strength of the medium. The parameter may be measured in the upper compartment 110a, in the lower compartment 110b or in the upper and the lower compartments (110a, 110b).

The bioreactor may further comprise a temperature control device configured to heat and/or cool the medium in the culturing tank 110.

The bioreactor may further comprise a controller configured to control at least one physical or chemical parameter. Said physical or chemical parameter is preferably controlled according to the parameter measured by the at least one sensor or by the density measurement device.

For example, the controller may be configured to actuate the extraction device, for example the pump of the extraction device, to suck and extract the medium from the culturing tank 110 when the pH or the ionic strength of the medium is outside a predetermined range of values. The controller may be subsequently configured to actuate the filling device to inject fresh medium so that the value of the pH or the ionic strength of the medium in the culturing tank 110 recovers a value comprised in the predetermined range.

In another example, the controller is configured to control the temperature control device to heat and/or cool the medium in the culturing tank 110 according to the values measured by the temperature sensor.

In another example, the controller is configured to control the sliding of the filter according to the values measured by the density measurement device.

In another example, the controller is configured to control the lysing device and/or agitating device 180.

The controller may comprise predefined instructions, for example, a series of instructions for controlling the bioreactor 100 according to the different values measured by the sensors or the density measurement device. The controller may comprise a user interface, via which instructions can be entered by a user and/or information can be retrieved. The user interface includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system. The bioreactor 100 can be controlled by the controller based on a machine learning algorithm or an artificial intelligence system.

This disclosure also relates to a method for cell culturing using the bioreactor 100 described above. Main steps of the method are represented in figure 4.

The method comprises a first the steps of positioning the filter 120 so that the upper compartment 110a has a volume of more than one twentieth of the volume of the lower compartment 110b. In other words, the lower compartment 110b has a volume lower than 95% of the capacity of the culturing tank 110 at this step. Preferably, the lower compartment 110b has a volume ranging from 10% to 40% of the capacity of the culturing tank 110 at this step leading to a volume of the upper compartment 110a ranging from 1.5 to 9 times the volume of the lower compartment 110b. Preferably, the minimum volume of the lower compartment 110b at the cell seeding is 50 milliliters.

Then, the lower compartment 110b is filled with a medium. Preferably, the medium fills the capacity of the culturing tank 110 leading to totally filled lower compartment 110b and upper compartment 110a. Alternatively, the culturing tank 110 may be at least partially filled with the medium before positioning the filter 120 so that at least the lower compartment 110b is filled. The medium is preferably a medium 310 comprising at least one growing agent allowing cell growth.

At least one cell 200 to be cultured is deposited (seeded) in the culturing tank 110. The cells 200 may be deposited in the bottom of the culturing tank 110 before placing the filter 120 in the culturing tank 110. Alternatively, the cells 200 may be deposited in the filled lower compartment 110b through an opening disposed in the filter 120 or in the outer wall 111 of the culturing tank 110.

The culturing tank 110 is then optionally hermetically sealed, for example by closing the top 112 with the top cover 113.

At this stage, cells 200 are growing in the lower compartment 110b as represented in figure 4A. Because of the cell growth, the density of cells increases in the lower compartment 110b. According to the increase of cell density, the filter 120 may be slid up towards the top 112 of the culturing tank 110 to increase the volume of the lower compartment 110b. For example, the filter 120 may be slid up to maintain the density of the cultured cells 200 in the lower compartment 110b lower than 10¹² cells/mL, preferably lower than 10⁸ cells/mL, even more preferably lower than 10⁵ cells/mL. For example, the density of the cultured cells is maintained lower than 10⁶ cells/mL for mammalian cells, lower than 10⁸ cells/mL for yeast cells and lower than 10⁷ cells/mL for bacteria. Preferably, the cell density is measured during the cell growth using the density measurement device. Before, during or after the slide up of the filter 120, medium 310 comprising growing agent(s) may be injected to ensure that the lower compartment 110b remains filled as represented in figure 4B.

During at least part of the cell growth, the medium may be agitated using the agitating device 180. For example, when using blades for agitating the medium, the rotational velocity of the blades may range from 20 to0 rounds per minute (rpm).

After a predetermined culturing time, at least part of the medium is refreshed. The predetermined culturing time may be determined by the cell density. A volume of medium to be refreshed is thus extracted and fresh medium is injected using the medium managing device.

To extract the medium, the filter 120 may be slid towards the bottom 115 of the culturing tank 110 to decrease the volume of the lower compartment 110b as represented in figure 4C. This allows to separate cells 200 from the medium without centrifugation. For example, the filter 120 may be slid until the volume of the lower compartment 110b is ranging from 20% to 40% of the capacity of the culturing tank 110. The filter 120 may be slid until the volume of the lower compartment 110b is lower than one fourth of the volume of the upper compartment 110a therefore leading to a volume of the lower compartment 110b of less than 30% of the capacity of the culturing tank 110. In another example, filter 120 is slid until the cell density reaches a predetermined target compression density. The predetermined target compression density may range from 10⁵ cells/mL to 10¹⁴ cells/mL, preferably from 10⁷ to 10¹² cells/mL. For example, the predetermined target compression density is ranging from 10⁷ cells/mL to 10⁸ cells/mL for mammalian cells, from 10⁸ cells/mL to 10⁹ cells/mL for yeast cells and from 10⁹ cells/mL to 10¹² cells/mL for bacteria. The medium is then extracted from the upper compartment 110a as represented in figure 4D. Preferably, the medium is extracted until the upper compartment 110a is empty. Fresh medium is then injected as shown in figure 4E. Preferably, fresh medium is injected to fill at least 80%, preferably at least 90% of the capacity of the culturing tank 110. The filter 120 may then be optionally slid towards the top 112 of the culturing tank 110 to decrease the volume of the upper compartment 110a while maintaining the lower compartment 110b filled as represented in figure 4F. For example, the filter 120 is slid until the density of the cultured cells 200 in the lower compartment 110b reaches a predetermined target expansion density. The predetermined target expansion density may range from 10³ cells/mL to 10¹² cells/mL, preferably from 10⁵ to 10⁸ cells/mL. For example, the predetermined target expansion density is ranging from 10⁵ cells/mL to 10⁶ cells/mL for mammalian cells, from 10⁷ cells/mL to 10⁸ cells/mL for yeast cells and from 10⁷ cells/mL to 10⁹ cells/mL for bacteria.

The medium extracted out from the upper compartment 110a may be the medium 310 comprising growing agent(s) and the fresh medium injected in the upper compartment may be a medium 310 also comprising growing agent(s), optionally the same growing agent(s), as in figure 4E. This allows to continue the growth of cells 200. Contrarily to adding only nutrients during the growth (fed-back), refreshing the medium by extraction and injection allows to prevent culture waste accumulation. This refreshing of medium (with or without sliding of the filter 120) may be repeated at least two times during the cell growth.

The medium extracted out the upper compartment may be the medium 310 comprising growing agent(s). Fresh medium injected to replace said extracted medium may be a medium 330 comprising transfection agent(s) and optionally chemical agent(s). The remaining medium in the lower compartment 110b and the fresh medium 330 may be mixed using the agitating device 180. This method reduces transfection costs in terms of both chemical agent(s)and transfection agent(s). Indeed, in order to carry out chemical transfection, it is necessary to obtain a large biomass, as dividing cells 200 tend not to retain the plasma transgene. Adding the transfection reagents directly to the culture medium as in the method known from the prior art results in very high reagent consumption. However, using the bioreactor 100 allows to reduce the volume of the lower compartment 110b using the filter 120. This leads to a cell concentration without need of any centrifugation and resuspending. The quantity of transfection and chemical agents using the bioreactor 100 is thus drastically reduced, with a significant impact on cost.

The method may further comprise a step of transfection of cells 200.

The transfection may comprise a first step of injecting at least one transfection agent and optionally chemical agent in the culturing tank 110.

Transfection may be performed only chemically using chemical agent(s) injected with the fresh medium 330.

Transfection may be performed only physically using the electroporation device 120.

Transfection may be performed chemically and physically using chemical agent(s) injected with the fresh medium 330 and the electroporation device 120.

The electroporation may be performed on cells 200 without any increase of the cell density.

Alternatively, to perform the electroporation, the filter 120 may be slid down towards the bottom 115 of the culturing tank 110 to decrease the volume of the lower compartment 110b. For example, the filter 120 may be slid until the volume of the lower compartment 110b is ranging from 20% to 40% of the capacity of the culturing tank 110. The filter 120 may be slid until the volume of the lower compartment 110b is lower than one fourth of the volume of the upper compartment 110a leading to a volume of the lower compartment 110b of less than 30% of the capacity of the culturing tank 110. In another example, filter 120 is slid until the cell density reaches a predetermined target electroporation density. The predetermined target electroporation density may range from 10⁵ cells/mL to 10¹⁴ cells/mL, preferably from 10⁶ to 10¹² cells/mL. For example, the predetermined target electroporation density is ranging from 10⁶ cells/mL to 10⁸ cells/mL for mammalian cells, from 10⁷ cells/mL to 10⁹ cells/mL for yeast cells and from 10¹⁰ cells/mL to 10¹² cells/mL for bacteria. The medium is then extracted from the upper compartment 110a. Preferably, the medium is extracted until the upper compartment 110a is empty. If a chemical agent(s) is used, fresh medium 330 comprising these chemical agent(s) may be injected, preferably one or two volumes of fresh medium 330 comprising these chemical agent(s) is injected, in the upper compartment 110 and mixed in the lower compartment 110b, preferably before sliding the filter 120 down for electroporation.

Advantageously, the cultured cells 200 may be washed before electroporation. Indeed, washing allows to remove the salts which could cause arcing during electroporation. To wash the cultured cells 200, after sliding down of the filter 120 and extracting the medium, a conditioning buffer containing no salts nor DNA may be injected as represented in figure 4G. One to five volumes of conditioning buffer may be injected. Preferably, conditioning buffer is injected to fill the upper compartment 110a. The filter 120 may be slid up the top 112 of the culturing tank 110 to increase the volume of the lower compartment 110b and mix the conditioning buffer with the medium remaining in the lower compartment 110b before sliding the filter 120 down towards the bottom 115 of the culturing tank 110 to perform the electroporation. The sliding up and down may be repeated to improve the medium mixing. The excess of medium in the upper compartment 110a may be extracted before the electroporation as represented in figure 4H. The extraction of the excess of medium is performed before injecting chemical agent(s).

In the embodiment wherein the electroporation device 120 comprises an electroporation chamber 162 disposed outside the culturing tank 110, cells 200 may be moved inside the electroporation chamber 162 as in figure 4I by using, for example, a peristaltic pump. Cells 200 are then electroporated and optionally move back to the lower compartment 110b. Optionally, fresh medium is then injected in the culturing tank 110. The fresh medium preferably comprises growing agent(s). Preferably, the fresh medium is injected to fill at least 80%, preferably at least 90% of the capacity of the culturing tank 110. The filter 120 may then be optionally slid towards the top 112 of the culturing tank 110 to decrease the volume of the upper compartment 110a while maintaining the lower compartment 110b filled, for example by injecting medium in the upper compartment 110a. For example, the filter 120 is slid until the density of the cultured cells in the lower compartment 110b reaches the predetermined target expansion density.

The method may further comprise a step of cell lysing. This step is preferably performed after the cell growth and transfection. In the embodiment wherein beads are used to grind cells 200, the beads may be disposed in the culturing tank 110 all along the cell growth or may be injected at the end of the cell growth.

### NUMERICAL REFERENCES

100 - Bioreactor / 110 - Culturing tank / 110a - Upper compartment / 110b - Lower compartment / 111 - Outer wall of the culturing tank / 112 - Top of the culturing tank / 113 - Top cover / 115 - Bottom of the culturing tank / 116 - Bottom cover / 120 - Filter / 122a - Upper part of the filter holder / 122b - Lower part of the filter holder / 160 - Electroporation device / 162 - Electroporation chamber / 170 - Actuator / 172 - Helical thread / 174 - External ring / 180 - Agitating device / 181 - Impeller / 182 - Shaft / 190a - Magnets / 190b - Magnetic elements / 200 - Cells / 310 - Medium comprising growing agent(s) / 330 - Medium comprising conditioning buffer / A - Tank axis

## Claims

1. A bioreactor (100) for cell culturing, the bioreactor (100) comprising:
- a culturing tank (110) configured to receive at least one cell (200) to be cultured in a medium, the culturing tank (110) extending along a tank axis (A),
- a filter (120) disposed in the culturing tank (110) so that the filter (120) separates the culturing tank into an upper compartment (110a) and a lower compartment (110b), the filter (120) being configured to confine the at least one cell (200) in the lower compartment (110b), the filter (120) being configured to slide along the tank axis (A) thereby modifying volumes of the upper compartment (110a) and the lower compartment (110b),
- an extraction device in fluidic communication with the culturing tank (110), the extraction device being configured to extract the medium out of the upper compartment (110a) of the culturing tank (110),
- a filling device in fluidic communication with the culturing tank (110), the filling device being configured to inject fresh medium in the upper compartment (110a) of the culturing tank (110), and
- an agitating device (180) disposed in the lower compartment (110b) and configured to agitate the medium, the agitating device (180) being preferably configured to slide along the tank axis (A).

2. The bioreactor (100) for cell culturing according to claim **1**, further comprising an actuator (170) configured to slide the filter (120) along the tank axis (A).

3. The bioreactor (100) for cell culturing according to any one of claims **1** to **2**, wherein the culturing tank (110) has a capacity ranging from 50 milliliters to 30 liters, preferably ranging from 1 liter to 25 liters, more preferably ranging from 5 liters to 20 liters.

4. The bioreactor (100) for cell culturing according to any one of claims **1** to **3**, wherein the extraction device is configured to extract the medium out of the upper compartment (110a) when the upper compartment (110a) has a volume of more than twice a volume of the lower compartment (110b), preferably more than four times the volume of the lower compartment (1 10b), even more preferably more than 9 times the volume of the lower compartment (110b).

5. The bioreactor (100) for cell culturing according to claim **4**, wherein the filling device is configured to inject the fresh medium in the upper compartment (110a) when the upper compartment (110a) has a volume of more than twice a volume of the lower compartment (110b), preferably more than four times the volume of the lower compartment (110b), even more preferably more than 9 times the volume of the lower compartment (110b).

6. The bioreactor (100) for cell culturing according to any one of claims **1** to **5** further comprising an electroporation device (160) in fluidic communication with the culturing tank (110).

7. The bioreactor (100) for cell culturing according to any one of claims **1** to **6** further comprising a density measurement device in optical contact with the lower compartment, the density measurement device being configured to measure the density of the cultured cells, preferably the density measurement device is an optical device having a working wavelength ranging from 400 nm to 800 nm.

8. The bioreactor (100) for cell culturing according to any one of claims **1** to **7** further comprising at least one sensor configured to measure at least one parameter of the medium, the measured parameter being preferably a temperature or a pH.

9. The bioreactor (100) for cell culturing according to any one of claims **1** to **8** further comprising a lysing device configured to lyse the cultured cells.

10. The bioreactor (100) for cell culturing according to claim **9**, wherein the lysing device comprises an ultrasonic device.

11. A method for cell culturing the method comprising:
- providing a bioreactor (100) for cell culturing according to any one of claims **1** to **10**,
- disposing at least one cell (200) to be cultured in the culturing tank (110) comprising a medium,
- agitating the medium with the agitating device (180),
- after a predetermined culturing time, refreshing the medium by:
∘ sliding the filter (120) from a position wherein the upper compartment (110a) has a volume of more than one tenth of a volume of the lower compartment (110b) to decrease the volume of the lower compartment (110b) until the volume of the lower compartment (110b) is lower than one fourth of the volume of the upper compartment (110a),
∘ extracting, with the extraction device, the medium out of the upper compartment (110a),
∘ injecting, with the filling device, a fresh medium in the upper compartment (110a), and
∘ sliding the filter (120) to decrease the volume of the upper compartment (110a) so that a density of the cultured cells in the lower compartment (110b) is lower than a predetermined target expansion density while maintaining the lower compartment (110b) filled.

12. The method for cell culturing according to claim **11** wherein the medium comprised in the culturing tank (110) comprises at least one growing agent and the fresh medium comprises at least one growing agent.

13. The method for cell culturing according to claim **11** wherein the medium filling the lower compartment (110b) comprises at least one growing agent and the fresh medium comprises at least one transfection agent.

14. The method for cell culturing according to any one of claims **11** to **13** further comprising:
- transfecting at least part of the cultured cells immersed in a medium comprising at least one transfection agent, transfecting comprising:
∘ sliding the filter (120) to decrease the volume of the lower compartment (110b) until the volume of the lower compartment (110b) is lower than one fourth of the volume of the upper compartment (110a),
∘ extracting, with the extraction device, the medium out of the upper compartment (110a),
∘ injecting, with the filling device, a conditioning buffer in the upper compartment (110a),
∘ sliding the filter (120) to decrease the volume of the upper compartment (110a),
∘ sliding the filter (120) to decrease the volume of the lower compartment (110b) until the volume of the lower compartment (110b) is lower than one fourth of the volume of the upper compartment (110a),
∘ extracting, with the extraction device, the medium out of the upper compartment (110a), and
ο performing an electroporation by generating at least one electrical pulse on the at least part of the cultured cells.

15. The method for cell culturing according to any one of claims **11** to **14** further comprising, after disposing at least one cell (200) and before refreshing the medium, sliding the filter (120) to decrease the volume of the upper compartment (110a) so that a density of the cultured cells in the lower compartment (120b) is lower than a predetermined target expansion density while maintaining the lower compartment (120b) filled.

## Amended claims

### Amended claims in accordance with Rule 137(2) EPC.

1. A bioreactor (100) for cell culturing, the bioreactor (100) comprising:
- a culturing tank (110) configured to receive at least one cell (200) to be cultured in a medium, the culturing tank (110) extending along a tank axis (A),
- a filter (120) disposed in the culturing tank (110) so that the filter (120) separates the culturing tank into an upper compartment (110a) and a lower compartment (110b), the filter (120) being configured to confine the at least one cell (200) in the lower compartment (110b), the filter (120) being configured to slide along the tank axis (A) thereby modifying volumes of the upper compartment (110a) and the lower compartment (110b),
- an extraction device in fluidic communication with the culturing tank (110), the extraction device being configured to extract the medium out of the upper compartment (110a) of the culturing tank (110),
- a filling device in fluidic communication with the culturing tank (110), the filling device being configured to inject fresh medium in the upper compartment (110a) of the culturing tank (110),
- an agitating device (180) disposed in the lower compartment (110b) and configured to agitate the medium, the agitating device (180) being configured to slide along the tank axis (A), and
- a lysing device configured to lyse the cultured cells and/or an electroporation device (160) in fluidic communication with the culturing tank (110).

2. The bioreactor (100) for cell culturing according to claim 1, further comprising an actuator (170) configured to slide the filter (120) and/or the agitating device (180) along the tank axis (A).

3. The bioreactor (100) for cell culturing according to claim 2, wherein the actuator (170) comprises one of the following:
- at least one helical thread (172) disposed inside the culturing tank (110 parallelly to the tank axis (A), the filter (120) and/or the agitating device (180) comprising a nut corresponding to the helical thread (172);
- at least one helical thread (172) disposed outside the culturing tank (110) parallelly to the tank axis (A), each helical thread (172) comprising a nut comprising a magnet, the filter (120) and/or the agitating device (180) comprising a magnetic element corresponding to the magnet of the helical thread (172);
- at least one helical thread (172) disposed outside the culturing tank (110) parallelly to the tank axis (A), an external ring (174) disposed outside the culturing tank (110) and cooperating with the helical threads (172), the filter (120) and/or the agitating device (180) comprising magnetic elements (190b) corresponding to magnets (190a) disposed on the external ring (174);
- a pinion meshes with a rack attached to the filter (120) and/or the agitating device (180); or
- a cable or belt wound around a pulley, the filter (120) and/or the agitating device (180) being attached to the cable or the belt.

4. The bioreactor (100) for cell culturing according to any one of claims 1 to 3, wherein the filling device comprises at least one medium reservoir fluidically connected to the culturing tank (110).

5. The bioreactor (100) for cell culturing according to claim 4, wherein each medium reservoir is fluidically connected to the culturing tank (110) via either:
- a filling channel wherein a first extremity is disposed in the medium reservoir and a second extremity is disposed in the upper compartment (110a); or
- several inlets are disposed though an outer wall (111) of the culturing tank (110), each inlet being connected to the medium reservoir via the filling channel and a valve.

6. The bioreactor (100) for cell culturing according to any one of claims 1 to 5, wherein the extraction device comprises a pump fluidically connected to the culturing tank (110).

7. The bioreactor (100) for cell culturing according to claim 6, wherein the pump is connected to the culturing tank (110) via either:
- an extraction channel wherein a first extremity is connected to the pump and a second extremity is disposed in the upper compartment 110a; or
- several outlets are disposed though an outer wall (111) of the culturing tank (110), each outlet being connected to the pump via the extraction channel and a valve.

8. The bioreactor (100) for cell culturing according to any one of claims 1 to 7, wherein the agitating device comprises either:
- a plurality of beads; or
- an impeller (181) comprising at least one blade, the impeller (181) being mounted on a shaft (182) configured to rotate the at least one blade

9. The bioreactor (100) for cell culturing according to any one of claims 1 to 8, wherein the culturing tank (110) has a capacity ranging from 50 milliliters to 30 liters, preferably ranging from 1 liter to 25 liters, more preferably ranging from 5 liters to 20 liters.

10. The bioreactor (100) for cell culturing according to any one of claims 1 to 9, wherein the extraction device is configured to extract the medium out of the upper compartment (110a) when the upper compartment (110a) has a volume of more than twice a volume of the lower compartment (110b), preferably more than four times the volume of the lower compartment (110b), even more preferably more than 9 times the volume of the lower compartment (110b).

11. The bioreactor (100) for cell culturing according to claim 10, wherein the filling device is configured to inject the fresh medium in the upper compartment (110a) when the upper compartment (110a) has a volume of more than twice a volume of the lower compartment (110b), preferably more than four times the volume of the lower compartment (110b), even more preferably more than 9 times the volume of the lower compartment (110b).

12. The bioreactor (100) for cell culturing according to any one of claims 1 to 11 further comprising a density measurement device in optical contact with the lower compartment, the density measurement device being configured to measure the density of the cultured cells, preferably the density measurement device is an optical device having a working wavelength ranging from 400 nm to 800 nm.

13. The bioreactor (100) for cell culturing according to any one of claims 1 to 12 further comprising at least one sensor configured to measure at least one parameter of the medium, the measured parameter being preferably a temperature or a pH.

14. The bioreactor (100) for cell culturing according to claim 13 further comprising a controller configured to control at least one physical or chemical parameter according to the parameter measured by the at least one sensor.

15. The bioreactor (100) for cell culturing according to claim 14 comprising the lysing device, wherein the lysing device comprises an ultrasonic device.
